# EUROPEAN PATENT APPLICATION

(11) **EP 1 772 730 A1**
(43) Date of publication of application: **11.04.2007**
(21) Application number: 05767304.8
(22) Date of filing: 28.07.2005
(51) Int. Cl.: G01N 31/00, G01N 31/22

(54) **REAGENT FOR LEAD ASSAY**

(30) Priority: 29.07.2004 US 591865 P
(71) Applicant: Kowa Co., Ltd., Nagoya-shi, Aichi 460-8625 (JP)
(72) Inventor: ASANO, Takaharu, 3050074 (JP); HARADA, Naoki, 3001217 (JP)
(74) Representative: Hartz, Nikolai
(86) International application number: PCT/JP2005/013823
(87) International publication number: WO 2006/011549

(57) **Abstract**

A reagent for lead assay whereby a lead concentration can be accurately assayed even if an analyte contains calcium ions, and a method for assaying the lead concentration using the reagent for lead assay are provided. The reagent for lead assay contains a porphyrin nucleus-incorporated polymer, which is obtained by radical copolymerization of a porphyrin compound with a vinylene monomer, and calcium ions.

## Description

### Technical Field

The present invention relates to a reagent for lead assay containing a porphyrin nucleus-incorporated polymer and calcium ions, and a method for assaying the lead concentration using the reagent for lead assay.

### Background Art

Lead has long been used for water pipes since it is a soft and easily workable metal. In recent years, it has been used for particular parts such as ones placed before and after water meters. Although lead was once said to be hardly soluble due to an oxide film formed on the surface thereof, the elution thereof has recently been seen as a problem, and water service businesses have begun to switch lead pipes to stainless steel pipes, etc. For preventing the elution of lead from incinerated ash and fly ash exhausted from incineration plants of urban garbage and industrial wastes, studies on developments of elution preventive agents and treatment methods are being performed. Lead is known to cause neural disorders and symptoms of intoxication such as anaemia, headache, anorexia and lead colic.
In view of the above-described situation, methods for simply, rapidly and precisely assaying lead contained in tap water, well water, river, wastewater, waste liquid and the like, are desired.

As a simple method for assaying lead in water, assay by absorptiometry is known. Porphyrin compounds are used as a colorimetric reagent in absorptiometry. A porphyrin nucleus-incorporated polymer obtained by radical copolymerization of the porphyrin compounds represented by thelaterdescribedformulas (1) and (2) with a vinylene monomer is known to be usable as a reagent for assay of a trace amount of a metal and to be capable of assaying the lead concentration (Patent Document 1).
[Patent Document 1] U.S. Patent No. 6437067

The present inventors assayed the lead concentration contained in waste waters, etc. using the porphyrin nucleus-incorporated polymers, and have found that there arises such a problem that when calcium ions coexist, the absorbance at near 465 nm, which is an absorption peak for a lead-porphyrin nucleus-incorporated polymer complex, increases, and the assay value becomes higher than the actual lead concentration.

Accordingly, it is an object of the present invention to provide a reagent for lead assay and a method for assaying the lead concentration using the reagent for lead assaywhereby when the lead concentration is assayed using the porphyrin nucleus-incorporated polymers, the lead concentration can accurately be assayed even if the analyte contains calcium ions.

### Disclosure of the Invention

Under such situations, the present inventors have found that by preliminarily adding calcium ions to an aqueous solution of a porphyrin nucleus-incorporated polymer, the lead concentration is precisely assayed without being affected by calcium ions present in an analyte. This finding has led to the completion of the present invention.

Specifically, the present invention provides a reagent for lead assay containing a porphyrin nucleus-incorporated polymer obtained by radical copolymerization of a porphyrin compound represented by the following formula (1) or (2):

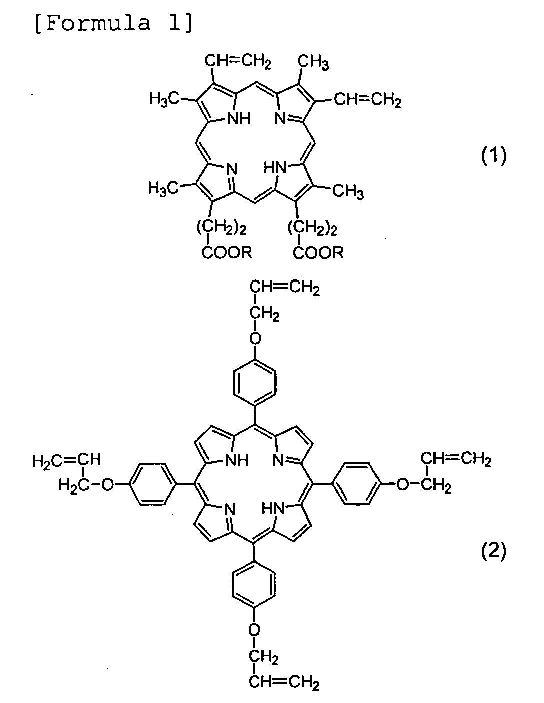

(wherein R represents a hydrogen atom or an alkyl group having 1 to 6 carbon atoms)
with a vinylene monomer and containing calcium ions.
The present invention further provides a method for assaying the lead concentration using the reagent for lead assay containing the porphyrin nucleus-incorporated polymer and calcium ions.

The reagent for lead assay of the present invention is capable of precisely assaying the lead concentration without being affected by calcium ions present in an analyte, and is useful as a reagent for lead assay of waste waters, waste liquids, etc.

### Brief Description of the Drawings

Figure 1 is a diagram showing differential spectrums when an analyte water 1 was assayed using reagents for lead assay; and
Figure 2 is a diagram showing relations between absorbance differences and calcium concentrations present in analyte waters 1 to 9 when the absorbance difference at 465 nm was set at 1.

### Best Mode for Carrying Out the Invention

A porphyrin nucleus-incorporated polymer obtained by radical copolymerization of a porphyrin compound used in the present invention represented by the following formula (1) or (2) :

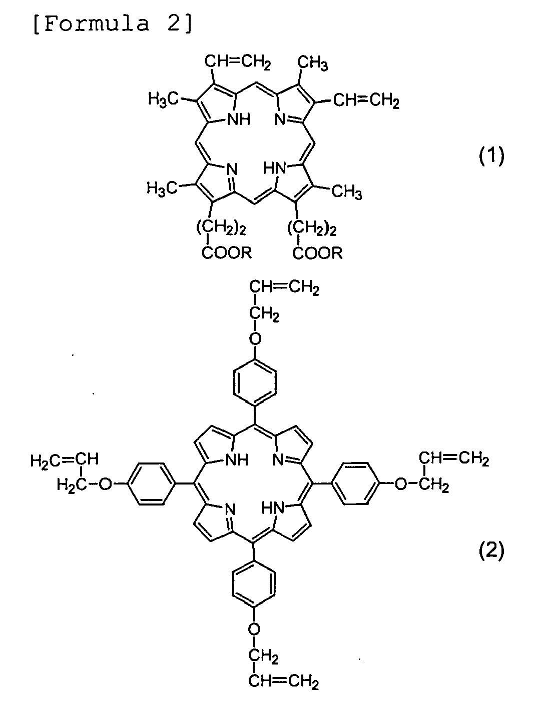

(wherein R represents a hydrogen atom or an alkyl group having 1 to 6 carbon atoms)
with a vinylene monomer, is manufactured by the method described in U.S. Patent No.6437067. Here, the two R groups in the formula (1) may be the same or different.

A porphyrin compound represented by the formula (1) or (2) constituting a porphyrin nucleus-incorporated polymer is, for example, protoporphyrin IX, in which R in the formula (1) is a hydrogen atom, protoporphyrin IX dimethyl ester, in which R in the formula (1) is a methyl group, and 5, 10, 15, 20-tetrakis(4-(allyloxy)phenyl)-21H,23H-porphyri n represented by the formula (2). These porphyrin compounds are commercially available.

A porphyrin compound is preferably protoporphyrin IX, in which R in the formula (1) is a hydrogen atom, and is preferably an alkaline metal salt thereof, especially a disodium salt thereof on manufacturing a porphyrin nucleus-incorporated polymer.

Vinylene monomers constituting porphyrin nucleus-incorporated polymers include compounds having one vinylene group, specifically acrylamide, methacrylic acid, acrylic acid, 5-hexenoic acid, allylamine, 3-butenoic acid, β-methallylalcohol, allylalcohol, N,N-dimethylacrylamide, 1-vinylimidazole, 2-vinylpyridine, 4-vinylpyridine, allyl chloride, vinyl acetate, crotonamide, maleic acid, fumaric acid, crotonic acid, isocrotonic acid, trans-1,2-dichloroethylene, citraconic acid, mesaconic acid, angelic acid, tiglic acid and 2-methyl-2-butene. The vinylene monomer is preferably acrylamide, N,N-dimethylacrylamide, 1-vinylimidazole, vinylacetate and the like, especially preferably acrylamide and N,N-dimethylacrylamide.

The amount ratio of a porphyrin compound to a vinylene monomer (i.e., porphyrin compound: vinylene monomer) to be used is preferably 1:100 to 1:10,000 in terms of mass ratio, and the ratio is especially preferably 1:200 to 1:1,000.

The molecular weight of a porphyrin nucleus-incorporated polymer obtained by radical copolymerization of a porphyrin compound and a vinylene monomer is not especially limited, but is preferably 50,000 to 5,000,000 in terms of average molecular weight by light scattering in view of the precision in lead assay, and it is especially preferably 100,000 to 1,000,000.

If a compound having two or more vinylene groups which functions as a crosslinking agent is used as a vinylene monomer of constitutional unit of a porphyrin nucleus-incorporated polymer used in the present invention together with a vinylene monomer having one vinylene group, the polymer chain is three-dimensionally crosslinked. This structure allows the polymer to be molded into an optional shape, allowing the reagent for lead assay of the present invention to take shapes other than a liquid. The reagent for lead assay can be molded into optional shapes suitable for installing places and measuring systems, such aspaste-like, sheet-like, film-like, tube-like and bead-like shapes.

The compounds having two or more vinylene group which functions as a crosslinking agent include N,N'-methylenebisacrylamide, N,N'-bisacryloylcystamine, divinylbenzene, ethylene glycol diacrylate, tetramethylolmethane tetraacrylate and trimethylolpropane triacrylate, and are preferably N,N'-methylenebisacrylamide, N,N'-bisacryloylcystamine and the like.
The compound having two or more vinylene group which functions as a crosslinking agent is preferably contained in an amount of 0.001 to 0.4 mass times a compound having one vinylene group, further preferably 0. 02 to 0.2 mass times.

The content of a porphyrin nucleus-incorporated polymer in the reagent for lead assay is preferably 1 to 60 mass% in view of the precision on assaying the concentration of lead contained in a trace amount in an analyte, further preferably 5 to 50 mass%, especially preferably 10 to 30 mass%.

A calcium ion-supplying compound to generate calcium ions used in the reagent for lead assay of the present invention is not especially limited as long as the compound does not damage the advantages of the present invention and liberates calcium ions. The compound includes, for example, inorganic calcium salts such as calcium chloride, calcium sulfate, calcium nitrate, calcium carbonate, calcium hydroxide, calcium hydrogenphosphate and calcium phosphate, and organic calcium salts such as calcium acetate, calcium formate, calcium lactate, calcium gluconate, calcium benzoate, calcium isobutyrate, calcium propionate and calcium salicylate. The calcium ion-supplying compound is preferably an inorganic calcium salt, for example, calcium chloride, calcium sulfate and calcium nitrate, especially preferably calcium chloride.

The calcium ion concentration made to coexist with the reagent for lead assay is preferably 0.1 to 100 mmol/L, especially preferably 2 to 20 mmol/L.

In addition to the above-mentioned components and water, optional components used for assay of heavy metal ions in common solutions can be added, in the range of not damaging advantages of the present invention, to the reagent for lead assay of the present invention. Such optional components include a pH adjusting agent, a surfactant, a chelating agent and a masking agent.

The pH adjusting agent includes N-cyclohexyl-3-aminopropanesulfonic acid, N-cyclohexyl-2-hydroxy-3-aminopropanesulfonic acid, sodium hydrogencarbonate and sodium carbonate.

The surfactant includes a cationic surfactant, an anionic surfactant and a nonionic surfactant.

The chelating agent and the masking agent include a polyethyleneimine, citric acid, ethylenediaminetetraacetic acid (EDTA) and ethylene glycol tetraacetic acid (EGTA).

Assay using the reagent for lead assay of the present invention is performed preferably at pH of a mixture solution of the reagent for lead assay and an analyte of 9 to 12 (25°C), further preferably 10 to 11. Here, in the case of the reagent for lead assay in a solid state, the pH denotes a pH after the reagent components have eluted into the analyte. When pH of the mixture solution is out of this range, the pH is preferably adjusted with a pH adjusting agent so as to be in this range.

The reagent for lead assay of the present invention may take a liquid, a paste-like, a sheet-like, a film-like, a tube-like, a bead-like or other shapes, but is preferably of a liquid or a sheet-like shape in view of easy handling.

Assay of the lead concentration contained in an analyte using the reagent for lead assay of the present invention is performed, for example, using a calibration curve using a standard sample by absorptiometry.
The absorption wavelength in measurement by an absorptiometer is preferably 350 to 700 nm, further preferably 400 to 500 nm, especially preferably 465 nm.

In the lead concentration assay using the reagent for lead assay of the present invention, the calcium ion concentration in an analyte is preferably not more than 1,000 mmol/L, especially preferably not more than 300 mmol/L.

### Examples

Hereinafter, the present invention will be further in detail described by way of examples, but the present invention is not limited thereto.

### Reference Example 1 Synthesis of a porphyrin nucleus-incorporated polymer

182.4 mg of protoporphyrin IX disodium salt and 71.2 g of acrylamide were dissolved in dimethyl sulfoxide (DMSO) to make 980 mL of a solution. The solution was transferred to a three-neck flask; 20 mL of azobisisobutyronitrile (AIBN) of 500 mmol/L was added thereto; then the solution was subjected to bubbling by nitrogen gas for 10 min; and immediately the flask was stopped up. The three-neck flask was immersed in a water bath adjusted at 60°C to initiate polymerization. After 4 h, the obtained polymerized liquid was charged dropwise little by little in a large amount of methanol of a precipitant to isolate a porphyrin nucleus-incorporated polymer.

### Example 1 Preparation of a reagent for lead assay

The resultant porphyrin nucleus-incorporated polymer was dissolved little by little in MilliQ water ® and diluted such that the absorbance (blank: air) at 400 nmwhen the diluted solution was 10 times diluted was observed between 0.5 and 0.6. The porphyrin nucleus-incorporated polymer aqueous solution thus obtained and a solution, having a pH adjusted at 11 by addition of sodium hydroxide, of 3-cyclohexyl-1-propanesulfonicacid (CAPS) of 100mmo1/Lwere mixed in a volume ratio of 3: 2 to prepare a comparative reagent for lead assay (pH: 11). Further, the comparative reagent for lead assay and a calcium chloride solution of 1, 000 mmol/L were mixed in a volume ratio of 500:1 to prepare a reagent for lead assay (pH: 11) of the present invention.

### Example 2 Study on the influence of coexisting calcium

### (1) Analyte waters

A lead standard solution for atomic absorption (1,000 mg/L) was diluted with MilliQ water ® and adjusted at 2 µmol/L (=0.41 mg/L). To the 2-µmol/L lead aqueous solution, small amounts of a high-concentration calcium chloride (not more than 1 mass% to the total amount) were added to prepare mixture solutions of lead and calcium shown in Table 1 as analyte waters.

**[Table 1]**

| Analyte water | Lead (µmol/L) | Calcium (mmol/L) |
|---|---|---|
| 1 | 2 | 0 |
| 2 | 2 | 1 |
| 3 | 2 | 3 |
| 4 | 2 | 10 |
| 5 | 2 | 30 |
| 6 | 2 | 100 |
| 7 | 2 | 300 |
| 8 | 2 | 1000 |
| 9 | 2 | 3000 |

### (2) Measurements

250 µL of the reagent for lead assay or the comparative reagent for lead assay, and 250 µL of MilliQ water ® were mixed (pH: 11) in a 1 cm square cuvette, and heated at 75°C for 5 min; then, an absorption spectrum of the resultant solution was measured with air as the blank and used as a reference spectrum. The reagent for lead assay or the comparative reagent for lead assay, and each analyte water were similarly mixed (pH: 11), and heated; and an absorption spectrum was measured. To remove the influence of base line fluctuation and amplified vibration, the absorption spectrum was normalized (SNV transformation) using measured points from 370 to 600 nm, and the reference spectrum was subtracted from the absorption spectrum to produce a differential spectrum.

### (3) Results

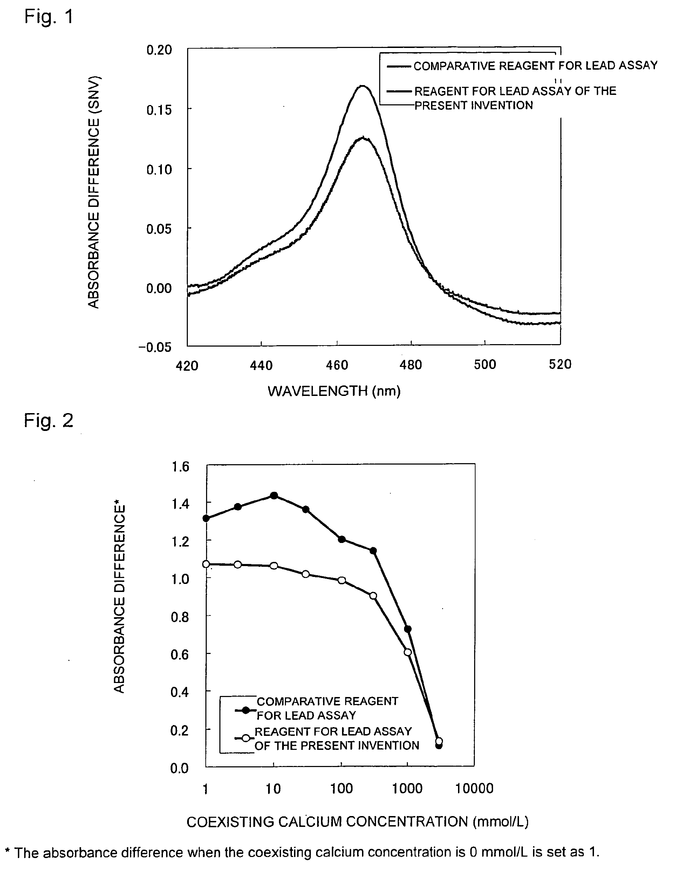

Figure 1 is the differential spectrums when the analyte water 1 was measured using the reagents for lead assay. The differential spectrum peak derived from lead is observed to be nearly at 465 nm for the either reagent. With the absorbance difference at 465 nm on this observation set to be 1, the relation between the respective calcium concentrations coexisting in the analyte waters and the absorbance differences is plotted in Figure 2. Figure 2 expresses the abscissa axis in logarithmic scale.
As is clear from Figure 2, when the reagent for lead assay not containing calcium is used, the coexistence of calcium of not more than 20 mmol/L increases the absorbance difference by not less than 40% at the maximum. By contrast, when the reagent for lead assay of the present invention is used, the increase in the absorbance difference due to the coexistence of calcium is 7% even at the maximum. When either of the reagent for lead assay of the present invention and the comparative reagent for lead assay is used and when a high-concentration calcium of not less than 100 mmol/L coexists, the absorbance difference is observed to have a tendency of decreasing according to the increase in the calcium concentration.
If the measurement error is allowed to 10%, while the calcium acceptable concentration in an analyte water when the reagent for lead assay not containing calcium is used is not more than 1 mmol/L, that when the reagent for lead assay of the present invention is used is not more than 300 mmol/L.

## Claims

1. A reagent for lead assay comprising a porphyrin nucleus-incorporated polymer and calcium ions, wherein the porphyrin nucleus-incorporated polymer is obtained by radical copolymerization of a porphyrin compound represented by the following formula (1) or (2): 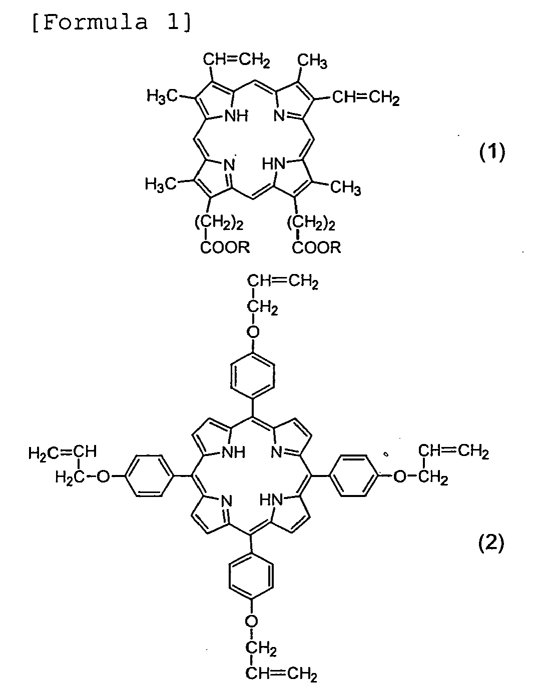 (wherein R is a hydrogen atom or an alkyl group having 1 to 6 carbon atoms)
with a vinylene monomer.

2. The reagent for lead assay according to claim 1, wherein the vinylene monomer constituting the porphyrin nucleus-incorporated polymer is selected from the group consisting of acrylamide, methacrylic acid, acrylic acid, 5-hexenoic acid, allylamine, 3-butenoic acid, β-methallylalcohol, allylalcohol, N,N-dimethylacrylamide, 1-vinylimidazole, 2-vinylpyridine, 4-vinylpyridine, allyl chloride, vinyl acetate, crotonamide, maleic acid, fumaric acid, crotonic acid, isocrotonic acid, trans-1, 2-dichloroethylene, citraconic acid, mesaconic acid, angelic acid, tiglic acid and 2-methyl-2-butene.

3. The reagent for lead assay according to claim 1, wherein the vinylene monomer constituting the porphyrin nucleus-incorporated polymer comprises a compound having one vinylene group and a compound having at least two vinylene groups, wherein both of which function as a crosslinking agent.

4. The reagent for lead assay according to claim 3, wherein the compound having at least two vinylene groups which functions as a crosslinking agent is selected from the group consisting of N,N'-methylenebisacrylamide, N,N'-bisacryloylcystamine, divinylbenzene, ethylene glycol diacrylate, tetramethylolmethane tetraacrylate and trimethylolpropane triacrylate.

5. The reagent for lead assay according to any one of claims 1 to 4, comprising calcium ions of 0.1 to 100 mmol/L.

6. A method for assaying a concentration of lead using the reagent for lead assay according to any one of claims 1 to 5.
